(19) Europäisches Patentamt
European Patent Office
Office européen des brevets

(11) Publication number: **0 042 075**
**B1**

(12) # EUROPEAN PATENT SPECIFICATION

(45) Date of publication of patent specification: **22.02.84**

(51) Int. Cl.³: **A 61 K 31/785,**
C 08 F 251/00, C 08 F 8/44

(21) Application number: **81103914.8**

(22) Date of filing: **21.05.81**

(54) **Copolymers having bactericidal activity, process for the preparation thereof and pharmaceutical compositions therefor.**

(30) Priority: **18.06.80 IT 2285080**

(43) Date of publication of application:
**23.12.81 Bulletin 81/51**

(45) Publication of the grant of the patent:
**22.02.84 Bulletin 84/8**

(84) Designated Contracting States:
**AT BE CH DE FR GB LI LU NL SE**

(56) References cited:
**FR - A - 2 109 104**
**FR - A - 2 416 910**
**US - A - 3 844 989**

**CHEMICAL ABSTRACTS, vol. 77, nr. 12, 18th September 1972, page 29, abstract 75875n, Columbus, Ohio, US, M.Yu. MAZOV: "Synthesis of cellulose graft copolymers with quarternary salts of 2-methyl-5-vinylpyridine"**

(73) Proprietor: **TEXCONTOR-ANSTALT**
**Vaduz (LI)**

(72) Inventor: **Maltz, Javier Esteban**
**Villanueva 1343**
**Buenos Aires (AR)**

(74) Representative: **Bianchetti, Giuseppe**
**Studio Consulenza Brevettuale Via Senato 24**
**I-20121 Milan (IT)**

**EP 0 042 075 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Copolymers having bactericidal activity, process for the preparation thereof and pharmaceutical compositions therefrom

The French Patent No. 2,416,910 discloses copolymers comprising a main chain of a natural polymer (cellulose) on which 2-methyl-5-vinyl-pyridine has been grafted, and the quarternization product of such a copolymer with dimethyl sulfate. The patent is silent concerning any pharmaceutical activity.

The present invention relates to copolymers and more specifically to copolymers of formula (I),

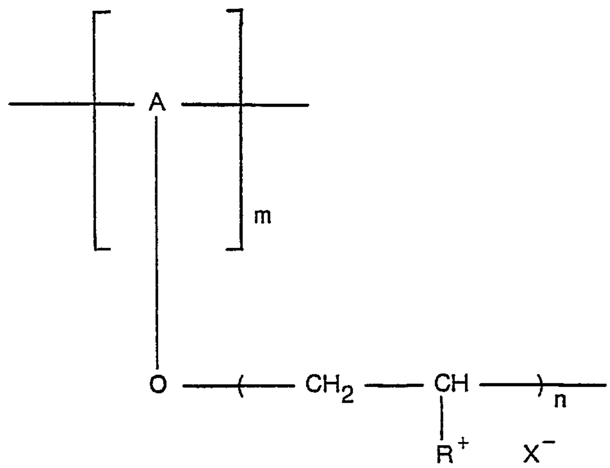

(I)

in which A is the monomeric unit of a polymer selected from the group consisting of oligosaccharides, polysaccharides, cellulosic derivatives and polyvinyl alcohol; m is the degree of polymerization; m and n are different one from the other, and m is a number between 10 and about 10,000 and n is a number between about 100 and about 250; R is a quaternary ammonium radical selected from the group consisting of

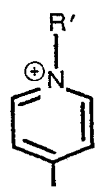 ; 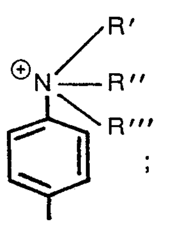 ; 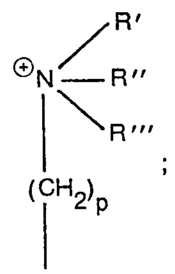 ; 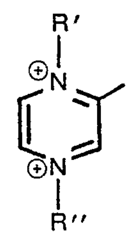

in which R′, R″, R‴ may be the same or different one from the other are $C_2$—$C_{16}$ alkyl or benzyl radicals and p is an integer between 0 and 10; $X^-$ is an anion selected from the group consisting of $Cl^-$, $Br^-$, $I^-$ $F^-$, or $HSO_4^-$.

Compounds of formula I unexpectedly have exhibited high bactericidal activity accompanied by a very low toxicity.

It is well known that disinfectants and antiseptics ordinarily called surface active agents which contain quaternary ammonium groups exhibits high bactericidal activity against both gram-positive and gram-negative organisms, as well as against funguses and viruses. The toxicity of these compounds varies from one product to the other: however, it is certain that the administration by the oral, rectal or vaginal route may cause serious damages particularly if the administration had been made erroneously. Particularly, when these substances are administered orally, they cause nausea and vomiting, and after they are absorbed by the body, they may produce muscular paralysis because of the possibility of blockage of the respiratory muscles with the result that asphyxia follows. The lethal dose of the most common quaternary surface active agents such as benzethonium chloride, benzalkonium chloride, methylbenzethonium chloride, cetylpyridinium chloride, has been estimated to be about 1—3 grams. The symptoms of poisoning are gastrointestinal irritation, dyspnea, cyanosis, collapse, convulsions, a state of dizziness, muscular weakness and finally death due to paralysis of the respiratory muscles.

In the concentrations used on the human skin, the solutions of the quaternary ammonium bases do not produce irritation but in some patients which are hypersensitive and after protracted use, they may produce phenomena of irritation and/or allergic reactions.

It has now been found surprisingly that the compounds of formula I in addition to exhibiting a very high activity, are also substantially devoid of toxicity both by the oral route and by application onto the skin as well as mucosae.

The synthesis of the products of the present application is carried out by means of a reaction of copolymerization which may be either ionic or of the free radical type by means of suitable initiators. The reaction of copolymerization may be carried out using as starting materials different monomeric units, thus obtaining copolymers which may be block copolymers or with an alternating or sequential arrangement. It is also possible to use as a starting material a pre-prepared polymer and then attach to it polymeric chain of different molecular weight of another functional monomer.

The monomeric unit which imparts to the product the chain

$$-\!\left(CH_2-\underset{\substack{|\\ R^+}}{CH}\right)\!-\quad X^-$$

may also be in the form of a quaternary salt and the quaternization may be carried out prior to the reaction of copolymerization or after the reaction of copolymerization.

One object of the present application is to provide the novel copolymers of formula I hereinabove. Another object is to provide a process for the preparation of the copolymers of formula I which involves grafting nitrogenous monomeric units, preferably 4-vinylpyridine onto oligosaccharides, polysaccharides, cellulosic derivatives, or polyvinyl alcohol.

The reaction of grafting is carried out by means of initiators for the purpose of forming suitable free radicals at a temperature lower than 40°C. For this reason, it is preferable to use redox initiator systems among which it is possible to mention the systems $Fe^{++}/H_2O_2$; $R—SH/H_2O_2$; $Na_2S_2O_3/H_2O_2$; $Ce^{4+}$ in solution of sulfuric or nitric acid, metallic salts of transition metals such as $V^{5+}$ and other similar salts.

Obviously, the products which are obtained depend upon the conditions used in the copolymerization reaction and specifically depend upon the quantity of the initiator used, the quantity of the monomer, the temperature and the reaction medium. It is, therefore, possible to obtain different quantities of synthetic polymers produced by an addition reaction with different molecular weight of the vinylpyridine chains which are grafted onto them. In general, the quantity of the polymer which is grafted varies between 10 and 60%, while the molecular weight is between 10,000 and 200,000. The quantity of the polymer which is grafted may be determined on the basis of the increase in weight of a known amount of oligosaccharide and polysaccharide after the reaction of copolymerization as soon as the homopolymer such as polyvinylpyridine has been eliminated by means of a selective extraction with a suitable solvent or by means of elemental analysis of the nitrogen which is contained in a known quantity in the copolymer.

The reaction of quaternization of the copolymer may be carried out using different alkylating agents such as ethyl bromide, propyl bromide, butyl bromide, etc., cetyl chloride, benzyl chloride, etc., in a variety of solvents.

The polyvinylpyridine, either as such or copolymerized is difficult to quaternize and the rate of quaternization decreases as the reaction proceeds. The choice of the reaction medium in which the reaction is carried out and the choice of the alkylating agent determine the extent of quaternization which may be achieved. Solvents having a high dielectric constant such as nitrobenzene, dimethylformamide, tetrahydrofuran, sulfolane, propylene carbonate have been found to be very suitable.

The quaternization reaction may be followed spectrophotometrically by infrared analysis during the course of reaction. Polyvinylpyridine exhibits a clear absorption band at 1600 $cm^{-1}$.

As soon as the reaction of quaternization proceeds, a new band at 1640 $cm^{-1}$ appears. The degree of quaternization is calculated on the basis of the results of elemental analysis and the amount of halogen ions and other negative ions.

The final products exhibit a degree of quaternization between 60 and 95%.

The following non-limitative examples are given by way of illustration of the invention.

Example 1

a) Natural maize starch, in the amount of 5 grams is dissolved in 50 ml of boiling water. To the solution, there is added concentrated nitric acid in such a manner as to obtain a concentration of 1.5 N, with respect to the acid. The mixture is deaerated to remove air for 10—20 minutes by passing nitrogen or argon through; then 10 ml of 4-vinylpyridine are added and subsequently a solution of cerium ammonium nitrate in nitric acid, so that the reaction system has a 0.01 M concentration with respect to $Ce^{4+}$. The reaction mixture is stirred for 12 hours at room temperature; then 400 ml of a mixture of 10% sodium hydroxide and 1,2-propanediol, in the ratio of 3:1 are added. The precipitate which is formed is centrifuged and washed with distilled water until the starch disappears in the washing water. Subsequently, the material is washed with methanol in order to eliminate the 4-polyvinylpyridine which has not been grafted onto the starch matrix. The product after filtering exhibits an infrared spectrum in KBr with the following characteristics bands: 1600 $cm^{-1}$, 1220 $cm^{-1}$, 1070 $cm^{-1}$ and 820 $cm^{-1}$. The elemental analysis has given the following values: $C = 52.78\%$; $H = 4.82\%$; $N = 7.34\%$.

b) The copolymer obtained as described in Example 1 in the amount of 7 grams, is suspended in

50 ml of methanol and treated with 10 grams of ethyl bromide. The reaction mixture is warmed under reflux for 120 hours, then the solvent and the excessive agent are evaporated up to a volume of about 15—20 ml. The material is then cooled to 0°C and the resulting quaternary salt which is formed is filtered.

I.R. KBr: 1600 $cm^{-1}$ band has disappeared and the 1640 $cm^{-1}$ band is now present. This band is characteristic of quaternized pyridine.

Analysis: Br = 39.80%.

The product which is obtained is designated hereinbelow for short with the symbol FCB 1010.

Example 2

a) Twenty grams of hydroxyethylcellulose of average molecular weight 40,000 are dissolved in 350 ml of water. To the solution there are added 20 grams of concentrated nitric acid and then after deaeration by means of nitrogen, 30 ml of 4-vinylpyridine followed by a solution of cerium ammonium nitrate in nitric acid in such a quantity to achieve a concentration of $Ce^{4+}$ of 0.01 M.

The reaction mixture is maintained under stirring for a period of 24 hours at 20°C and then 1000 ml of 10% sodium hydroxide are added. The precipitate which is formed, is filtered, washed with water and then with methanol.

I.R. KBr: 1600 $cm^{-1}$, 1200 $cm^{-1}$, 1045 $cm^{-1}$, 830 $cm^{-1}$.

Analysis: C = 42.46%; H = 5.76%; N = 3.05%.

b) The copolymer described in Example 2a, in the amount of 15 grams, is dissolved in 75 grams of nitrobenzene and quaternized in 25 grams of ethyl bromide at 100°C for a period of 48 hours. After the reaction is completed, the material is diluted with pentane and the quaternary salt which is formed is filtered and washed with a small amount of methanol.

I.R.: The 1600 $cm^{-1}$ band has disappeared and the 1640 $cm^{-1}$ band is now present.

Analysis: Br = 16.54%.

The material which is obtained will be referred to hereinbelow with the symbol FCB 1011.

*Microbiological Testing of the Antibacterial Activity of the Compounds FCB 1010 and FCB 1011*

1) Antibacterial activity as a function of the concentration of the products.

The products in the form of a whitish powder are dissolved in saline phosphate buffer prepared according to Sorensen of pH 7.2 in a concentration of 10% weight/volume which corresponds to 100 mg/ml.

For the purpose of testing the antibacterial activity of the products, 6 bacterial stocks purified by plaque clonation derived from human pathogenic germs maintained in the phase of logarithmic growth are used.

In particular, there are used the following organisms:

1) Gram — : Escherichia Coli (E.Coli)
Pseudomonas Aeruginosa (Ps.Aer.)
Proteus Vulgaris (Pr.Vulg.)
2) Gram + : Micrococcus Luteus (M.Lut.)
Streptococcus Fecalis (S.Fec.)
Staphylococcus Aureus (S.Aur.)

A liquid medium with ample spectrum of capacity of growth is used as the culture medium, that is Bactotryptic Soy Broth (Difco).

The activity of the products is determined using the concentrations of 0.1%, 0.01% and 0.001% in a liquid culture medium both for FCB 1010 and FCB 1011. For comparison purposes, there is used $\alpha$-(p-tolyl)dodecyltrimethylammonium methoxysulfate in the same concentrations.

For each dilution of the product there are utilized four test tubes each containing 20 ml of the liquid medium. The antibacterial activity is determined by the turbidometric test after 24 hours of incubation at 37°C. The test consists of determining the values of reduction of the light transmitted from the culture media which contain the micro-organisms under test and the products in different concentrations using as a control the same media together with the pharmaceutical substance being tested in the same concentration. The values are expressed in percentage of the cultures without addition of any antibacterial substance. For each test, there are used $7.5 \times 10^5$ live gram-negative bacteria and $9.2 \times 10^5$ live gram-positive bacteria.

*Results*

In Table 1 hereinbelow are reported the effects of FCB 1010 in different concentrations with respect to the organism under test. It is possible to observe that the product FCB 1010 in the concentration of 0.1% and 0.01% inhibits totally the growth of all bacteria tested. In the concentration of 0.001%, the substance exhibits an antibacterial activity, particularly with respect to *Escherichia Coli, Pseudomonas Aeruginosa* and *Micrococcus Luteus.*

In Table 2 hereinbelow, the antibacterial activity exhibited by the substances FCB 1011 is shown. Also, this substance prevents the antibacterial growth with all the organisms which have been studied up to a concentration of 0.01%, while in the concentration of 0.001, the activity decreases substantially, but the inhibitory effect of 50—60% is maintained.

The effect of $\alpha$-(p-tolyl)-dodecyltrimethylammonium methoxysulfate (Desogen), are tabulated in Table 3. They are substantially inferior with respect to the two preceding products. In fact, in the concentration of 0.01%, this substance does not prevent bacterial growth with any of the organisms tested.

TABLE 1
Antibacterial activity of the Product FCB 1010 at different concentrations with respect to several bacteria under test

| Test No. | Conc. of the Substance | E.Coli | Ps.Aer. | Pr.Vul. | M.Lut. | S.Fec. | S.Aur. |
|---|---|---|---|---|---|---|---|
| 1 | —— | 100% | 100% | 100% | 100% | 100% | 100% |
| 2 | 0.1% | 0% | 0% | 0% | 0% | 0% | 0% |
| 3 | 0.01% | 0% | 0% | 0% | 0% | 0% | 0% |
| 4 | 0.001% | 51% | 42.2% | 76.31% | 55% | 63% | 64% |

TABLE 2
Antibacterial activity of the Product FCB 1011 at different concentrations with respect to several bacteria under test

| Test No. | Conc. of the Substance | E.Coli | Ps.Aer. | Pr.Vul. | M.Lut. | S.Fec. | S.Aur. |
|---|---|---|---|---|---|---|---|
| 1 | —— | 100% | 100% | 100% | 100% | 100% | 100% |
| 2 | 0.1% | 0% | 0% | 0% | 0% | 0% | 0% |
| 3 | 0.01% | 0% | 0% | 0% | 0% | 0% | 0% |
| 4 | 0.001% | 59% | 44.3% | 66.4% | 61.3% | 58% | 61% |

TABLE 3
Activity of the Product Desogen at different concentrations with respect to the bacteria under test

| Test No. | Conc. of the Substance | E.Coli | Ps.Aer. | Pr.Vul. | M.Lut. | S.Fec. | S.Aur. |
|---|---|---|---|---|---|---|---|
| 1 | —— | 100% | 100% | 100% | 100% | 100% | 100% |
| 2 | 0.1% | 0% | 0% | 0% | 0% | 0% | 0% |
| 3 | 0.01% | 18% | 27% | 39% | 16% | 26% | 21% |
| 4 | 0.001% | 78% | 66% | 72% | 86% | 84% | 83% |

2) Antibacterial activity as a function of the concentrations of the products and their contact times with the bacteria.

The antibacterial activity as a function of the contact time of the surface active agents with the bacteria has also been investigated. The bacteria are suspended in water; FCB 1010, FCB 1011 and $\alpha$-(p-tolyl)-dodecyltrimethylammonium methoxysulfate have been used in concentrations of 0.1% and 0.01%. After 2, 5, 15 minutes of contact time, a sample of 0.1 ml has been placed into a medium of

liquid culture as described hereinabove for a period of 48 hours. Each test according to the different times and different concentrations has been carried out four times. The medium for the evaluation of the bacterial growth was the same as the method previously described. However, the results are expressed in a semi-quantitative manner in order to provide a better evaluation of the effective antibacterial activity (turbidimetric values of 1—30%+++; 30—60%++; 60—90%+). The results so obtained are reported in Table 4 hereinbelow using different contact times of the product with respect to the organisms with the two concentrations which have been studied.

It is evident that the products FCB 1010 and 1011 are active as antibacterial agents against all the organisms tested in the concentrations of 0.1% and 0.01% even after only two minutes of contact time.

The product used as a comparison exhibits an activity in the same order of magnitude against all bacteria except Pseudomonas Aeruginosa because in the case of this organism, the substance at the concentration of 0.01% does not exhibit a total bactericidal action.

TABLE 4

| Substances Tested | C.% | T.C. | E.Coli | Ps.Aer. | Pr.Vulg. | M.Lut. | S.Fec. | S.Aur. |
|---|---|---|---|---|---|---|---|---|
| FCB 1010 | 0.1 | 2 | — | — | — | — | — | — |
| | | 5 | — | — | — | — | — | — |
| | | 15 | — | — | — | — | — | — |
| | 0.01 | 2 | — | — | — | — | — | — |
| | | 5 | — | — | — | — | — | — |
| FCB 1011 | 0.1 | 2 | — | — | — | — | — | — |
| | | 5 | — | — | — | — | — | — |
| | | 15 | — | — | — | — | — | — |
| | 0.01 | 2 | — | — | — | — | — | — |
| | | 5 | — | — | — | — | — | — |
| | | 15 | — | — | — | — | — | — |
| DESOGEN | 0.1 | 2 | — | — | — | — | — | — |
| | | 5 | — | — | — | — | — | — |
| | | 15 | — | — | — | — | — | — |
| | 0.01 | 2 | — | + | — | — | — | + |
| | | 5 | — | + | — | — | — | — |
| | | 15 | — | + | — | — | — | — |

Disinfectant and antimycotic activity in "vivo"

a) The disinfectant activity in "vivo" has been studied in 40 patients affected by dermatosis of infective nature, generally pyogenic such as pyodermia, streptodermia, staphylodermia, acne, and in general inflamed prominent elevations of the skin. FCB 1010 and FCB 1011 have been applied on the skin of 20 patients both in the concentration of 1%, two or three times a day up to an improvement of the clinical condition. The therapeutic effect has been found to be optimum in 80% of the patients treated both in the case of FCB 1010 and FCB 1011. In addition, none of the treated patients exhibited

any reactions of cutaneous intolerance nor did any of the patients exhibit any reactions of allergic nature.

b) The antimycotic activity in "vivo" was determined with 30 patients affected by superficial cutaneous mycosis (epidermofizie) or different types both from the morphologic-clinical point of view as well as stage, caused by a variety of different funguses. Also, in this case, FCB 1010 and FCB 1011 were applied onto the skin in a concentration of 1%, two or three times a day to 15 patients up to an improvement of the clinical condition. This improvement was achieved very substantially in 86% of the individuals with FCB 1010 and 80% with FCB 1011.

During treatment, no secondary reactions, either at the cutaneous level or in general, were noted.

*Allergological Tests*

The allergological tests have been carried out with 60 individuals who had an allergic dermatosis and who had, therefore, high cutaneous activity, by application onto the skin.

FCB 1010 and FCB 1011 have been applied three times a day in different concentrations on an equal number of sections of the skin for a period of 30 days. The concentrations of the products selected for the test have been 4%, 2% and 1%. None of the patients manifested any symptoms of cutaneous intolerance connected with the treatment.

*Toxicity Tests*

FCB 1010 and FCB 1011 have been administered orally to Swiss rats and Sprague-Dawley rats in order to determine the acute toxicity, that is $DL_{50}$. The $DL_{50}$ has been found in both species to be greater than 3 g/kg. Another test has been carried out to determine the toxicity over an extended period of time with Beagle dogs for a period of four months. The tests have shown that with a daily dose of 1 or 2 g/kg, no change attributable to the products FCB 1010 and FCB 1011 has been found.

Another object of the invention is the preparation of pharmaceutical compositions for topical use, such as creams, ointments, lotions, etc., having antibacterial and antimycotic activity which contain as the active principle at least one of the copolymers of formula I, together with carriers and excipients conventionally used in the formulations of pharmaceutical compositions.

Still another object of the invention resides in the method of treatment of patients affected by dermatosis of infective nature, such as pyodermia, streptodermia, staphylodermia, acne and cutaneous mycosis by means of the compounds of formula I. Clearly, the invention covers all the operations required for the preparation of the copolymers, their purification, their formulation in pharmaceutical forms suitable for the administration and/or the final confection in containers suitable for the administration.

**Claims for the Contracting States: BE CH DE FR GB LI LU NL SE**

1. A copolymer having bactericidal activity of formula I

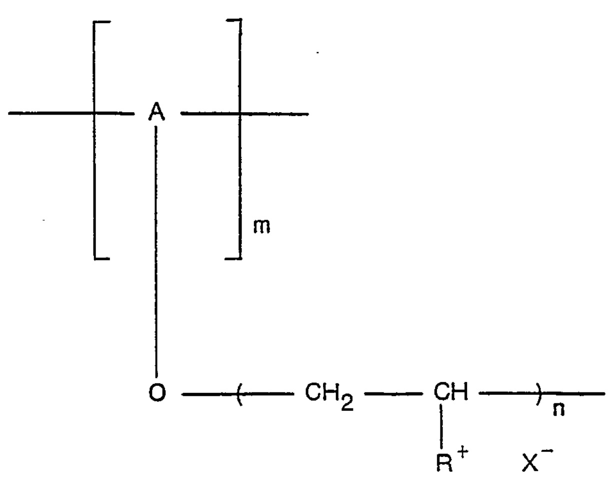

(I)

wherein A is the monomeric unit of a polymer selected from the group consisting of oligosaccharides, polysaccharides, cellulosic derivatives; and polyvinyl alcohol, m is the degree of polymerization; m and n are different one from the other, and m is a number between 10 and 10,000 and n is a number between 100 and 250;

R is a quaternary ammonium radical selected from the group consisting of

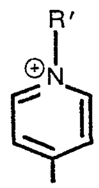 ; 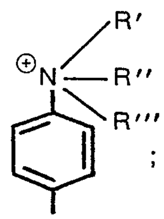 ; 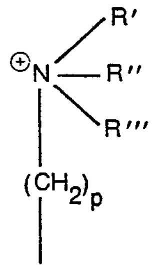 ; 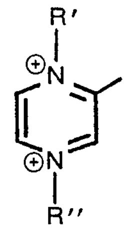

wherein R′, R″, R‴ are the same or different and are $C_2$—$C_{16}$ alkyl or benzyl and p is an integer between 0 and 10;
$X^-$ is an anion selected from the group consisting of $Cl^-$, $Br^-$, $I^-$, $F^-$, or $HSO_4^-$.

2. A copolymer according to claim 1 wherein A is the monomeric unit of starch.

3. A copolymer according to claim 1 wherein A is the monomeric unit of hydroxyethylcellulose.

4. A copolymer according to claim 2 wherein R is

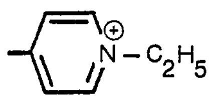

and $X^-$ is $Cl^-$, $Br^-$ or $I^-$.

5. A copolymer according to claim 3 wherein R is

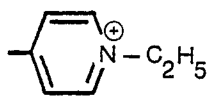

and X is $Cl^-$, $Br^-$ or $I^-$.

6. A pharmaceutical composition for topical use which contains as the active ingredient at least one compound according to claims 1—5 and at least one inert carrier and inert excipient.

**Claims for the Contracting State: AT**

1. A process for the preparation of copolymers having bactericidal activity of formula I

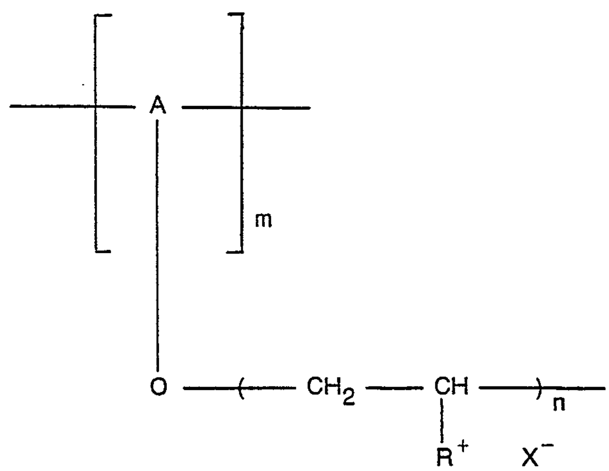

(I)

wherein A is the monomeric unit of a polymer selected from the group consisting of oligosaccharides, polysaccharides, cellulosic derivatives, polyvinyl alcohol, m is the degree of polymerization; m and n are different one from the other, and m is a number between 10 and 10,000 and n is a number between 100 and 250;

R is a quaternary ammonium radical selected from the group consisting of

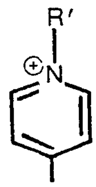 ; 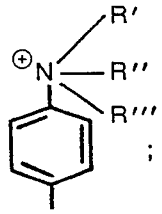 ; 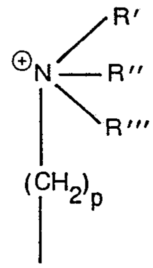 ; 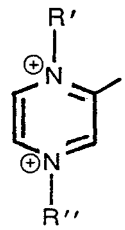

wherein R′, R″, R‴ are the same or different and are $C_2$—$C_{16}$ alkyl or benzyl and p is an integer between 0 and 10;
$X^-$ is an anion selected from the group consisting of $Cl^-$, $Br^-$, $I^-$, $F^-$, or $HSO_4^-$ comprising the grafting of monomeric units of the formula

$$\begin{array}{c} CH_2 = CH \\ | \\ R_1 \end{array}$$

wherein $R_1$ is a basic group which can be converted in $R^+X^-$, onto a polymeric chain of the formula

$$\left[ \begin{array}{c} A \\ | \\ OH \end{array} \right]_m$$

(wherein A and m have the above mentioned meanings), the reaction of grafting being carried out by means of initiators capable of forming free radicals at a temperature lower than 40°C; and the quaternization of the basic groups $R_1$ in the grafted copolymers so obtained.

2. Process according to claim 1, wherein the initiators are redox systems selected form the group consisting of $Fe^{++}/H_2O_2$; thiols/$H_2O_2$; $Na_2S_2O_3/H_2O_2$; $Ce^+$ in solution of sulfuric or nitric acid; salts of transition metals.

3. Process according to claims 1—2, wherein the quantity of the grafted chains of formula

$$\begin{array}{l} -\!\!\!+ CH_2 - CH +\!\!\!-_n \\ \qquad\quad | \\ \qquad\quad R^+ \quad X^- \end{array}$$

varies between 10 and 60% of the copolymer of formula (I), and their molecular weight is between 10,000 and 200,000.

4. Process according to claims 1—3, wherein A is the monomeric unit of starch.

5. Process according to claims 1—3, wherein A is the monomeric unit of hydroxyethylcellulose.

6. Process according to claims 1—5, wherein R is

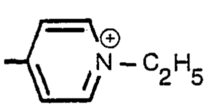

and X is $Cl^-$, $Br^-$ or $I^-$.

**Patentansprüche für die Vertragsstaaten: BE CH DE FR GB LI LU NL SE**

1. Copolymeres mit antibakterieller Aktivität der Formel I

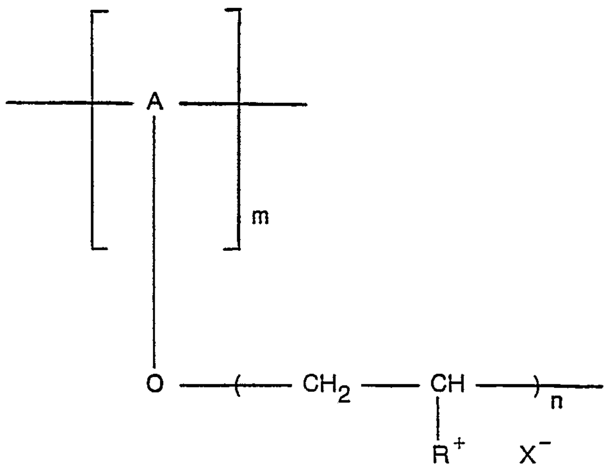

(I)

in der A die monomere Einheit eines Polymeren ist, das aus der Gruppe der Oligosaccharide, Polysaccharide, Cellulose-derivate und Polyvinylalkohol ausgewählt ist, m der Polymerisationsgrad ist; m und n voneinander verschieden sind und m eine Zahl zwischen 10 und 10 000 und n eine Zahl zwischen 100 und 250 ist; R ein quartärer Ammoniumrest ist, der ausgewählt ist aus der Gruppe von

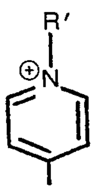 ; 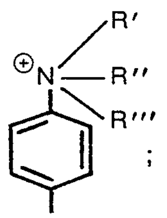 ; 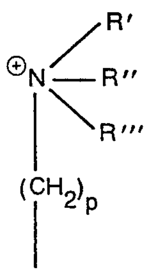 ; 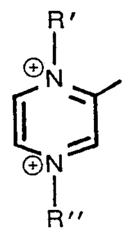

ist, worin R′, R″, R‴ gleich oder verschieden sind und $C_2$—$C_{16}$Alkyl- oder Benzylreste sind und p eine ganze Zahl zwischen 0 und 10 ist; $X^-$ ein Anion ausgewählt aus der Gruppe $Cl^-$, $Br^-$, $I^-$, $F^-$ oder $HSO_4^-$ ist.

2. Copolymeres nach Anspruch 1, dadurch gekennzeichnet, dass A die monomere Einheit von Stärke ist.

3. Copolymeres nach Anspruch 1, dadurch gekennzeichnet, dass A die monomere Einheit von Hydroxyethylcellulose ist.

4. Copolymeres nach Anspruch 2, dadurch gekennzeichnet, dass R

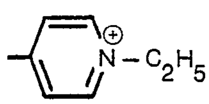

ist und dass $X^-$ $Cl^-$, $Br^-$ oder $I^-$ ist.

5. Copolymeres nach Anspruch 3 dadurch gekennzeichnet, dass R

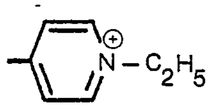

ist und dass $X^-$ $Cl^-$, $Br^-$ oder $I^-$ ist.

6. Pharmazeutische Zubereitungen für lokale Anwendung dadurch gekennzeichnet, dass Sie als Wirkstoff mindenstens eine Verbindung nach Anspruch 1—5 zusammen mit mindestens einer inerten Träger oder Verdünnungsmittel.

**Patentansprüche für den Vertragsstaat: AT**

1. Verfahren zur Herstellung von Copolymeren mit bakterizider Wirkung der allgemeinen Formel I

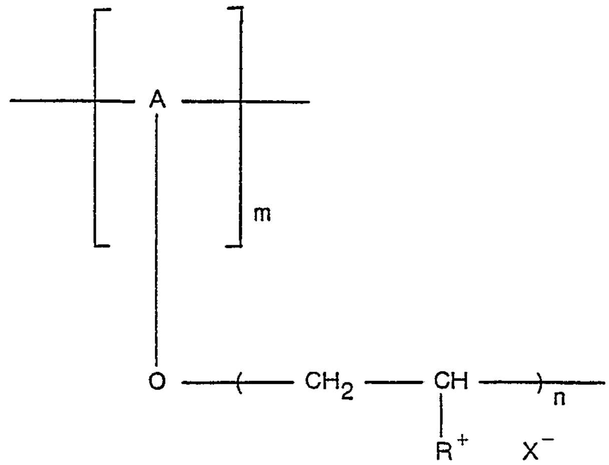

(I)

in der A die monomere Einheit eines Polymeren ist, das aus der Gruppe der Oligosaccharide, Polysaccharide, Cellulose-derivate und Polyvinylalkohol ausgewählt ist, wobei m der Polymerisationsgrad ist, m und n voneinander verschieden sind und wobei m eine Zahl zwischen 10 und 10 000 ist und n eine Zahl zwischen 100 und 250 ist, R ein quarterer Ammoniumrest ist, der ausgewählt ist aus den Gruppen

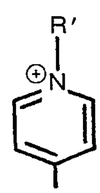 ; 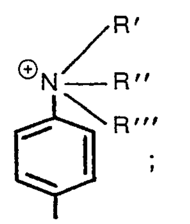 ; 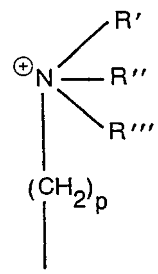 ; 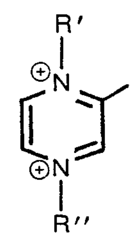

in denen R′, R″, R‴ gleich oder verschieden sind und $C_2$—$C_{16}$ Alkylreste oder der Benzylreste ist und p eine ganze Zahl zwischen 0 und 10 ist, $X^-$ ein Anion aus der Gruppe von $Cl^-$, $Br^-$, $I^-$, $F^-$ oder $HSO_4^-$ ist, umfassend das Aufpropfen der monomeren Einheiten der Formel

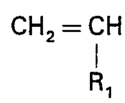

in der $R_1$ eine basische Gruppe ist, die in $R^+X^-$ umgewandelt werden kann, auf einer polymeren Kette der allgemeinen Formel

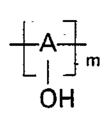

(in der A und m die vorstehenden Bedeutungen haben), wobei die Reaktion des Aufpropfens mit Hilfe von Initiatoren, die bei einer Temperatur von weniger als 40°C freie Radikale liefern, durchgeführt wird, und die Quaternisierung der basischen Gruppen $R_1$ in den so erhaltenen aufgepropften Copolymeren.

2. Verfahren nach Anspruch 1, dadurch gekennzeichnet, daß die Initiatoren Redoxsysteme sind, die ausgewählt sind aus den Gruppen $Fe^{++}/H_2O_2$, Thiole/$H_2O_2$, $Na_2S_2O_3/H_2O_2$, $Ce^+$ in Lösung von Schwefel- oder Salpetersäure, Salze der Übergangsmetalle.

3. Verfahren nach Anspruch 1 oder 2, dadurch gekennzeichnet, daß die Menge der aufgepfropften Ketten der allgemeinen Formel

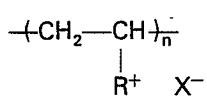

variiert zwischen 10 und 60% des Copolymeren der Formel (I) und daß ihr Molekulargewicht zwischen 10 000 und 200 000 liegt.

4. Verfahren nach Anspruch 1, 2 oder 3, dadurch gekennzeichnet, daß A die monomere Einheit von Stärke ist.

5. Verfahren nach Anspruch 1, 2 oder 3, dadurch gekennzeichnet, daß A die monomere Einheit von Hydroxyethylcellulose ist.

6. Verfahren nach einem der Ansprüche 1 bis 5, dadurch gekennzeichnet, daß R

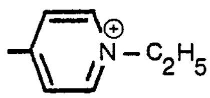

ist und X $Cl^-$, $Br^-$ oder $I^-$ ist.

**Revendications pour les Etats contractants: BE CH DE FR GB LI LU NL SE**

1. Copolymère possédant une activité bactéricide de formule I:

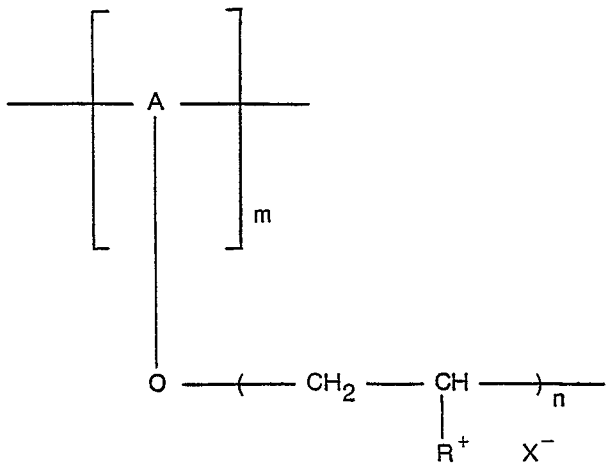

(I)

dans laquelle A est un motif monomère d'un polymère choisi dans le groupe comprenant les oligosaccharides, les polysaccharides, les dérivés cellulosiques et l'alcool polyvinylique, *m* est le degré de polymérisation; *m* et *n* sont différents l'un de l'autre, *m* est un nombre compris entre 10 et 10.000 et *n* est un nombre compris entre 100 et 250; R est un radical ammonium quaternaire choisi dans le groupe comprenant

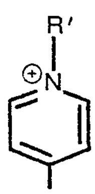 ; 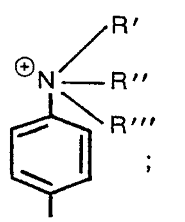 ; 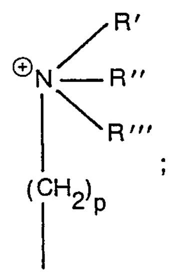 ; 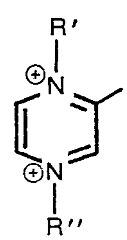

dans lesquels R′, R″ et R‴ sont identiques ou différents et désignent un radical alkyle en $C_2$—$C_{16}$ ou benzyle et *p* est un nombre entier compris entre 0 et 10; $X^-$ est un anion choisi dans le groupe comprenant $Cl^-$, $Br^-$, $I^-$, $F^-$ et $HSO_4^-$.

2. Copolymère selon la revendication 1 dans lequel A est le motif monomère de l'amidon.

3. Copolymère selon la revendication 1 dans lequel A est le motif monomère de l'hydroxyéthylcellulose.

4. Copolymère selon la revendication 2 dans lequel R est

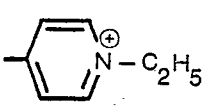

et $X^-$ est $Cl^-$, $Br^-$ ou $I^-$.

5. Copolymère selon la revendication 3 dans lequel R est

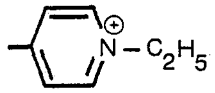

et $X^-$ est $Cl^-$, $Br^-$ ou $I^-$.

6. Composition pharmaceutique pour usage local qui contient à titre d'ingrédient actif, au moins un composé selon les revendications 1 à 5 et au moins un véhicule inerte et un excipient inerte.

**Revendications pour l'Etat contractant: AT**

1. Procédé pour la préparation d'un copolymère possédant une activité bactéricide de formule I

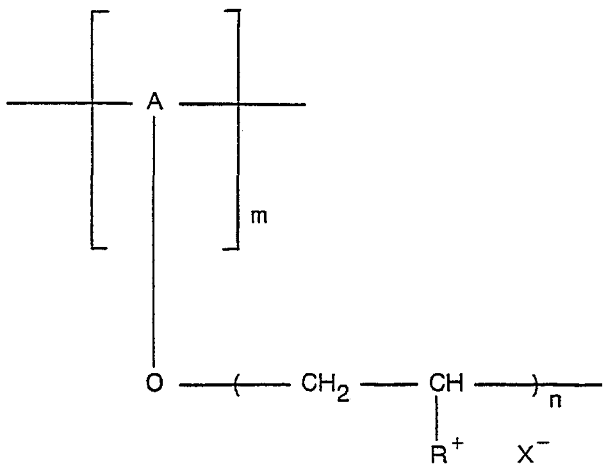

(I)

dans laquelle A est un motif monomère d'un polymère choisi dans le groupe comprenant les oligosaccharides, les polysaccharides, les dérivés cellulosiques et l'alcool polyvinylique, *m* est le degré de polymérisation; *m* et *n* sont différents l'un de l'autre, *m* est un nombre compris entre 10 et 10.000 et *n* est un nombre compris entre 100 et 250; R est un radical ammonium quaternaire choisi dans le groupe comprenant

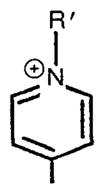 ; 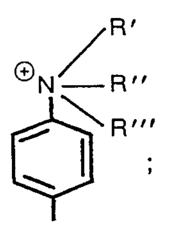 ; 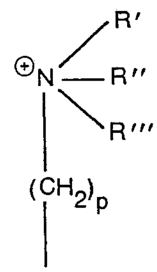 ; 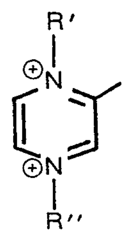

dans lesquels R', R'' et R''' sont identiques ou différents et désignent un radical alkyle en $C_2$—$C_{16}$ ou benzyle et *p* est un nombre entier compris entre 0 et 10; $X^-$ est un anion choisi dans le groupe comprenant $Cl^-$, $Br^-$, $I^-$, $F^-$ et $HSO_4^-$, consistant à greffer les motifs monomères de formule

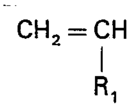

dans laquelle $R^1$ est un groupe basique pouvant être converti en $R^+X^-$, sur une chaîne polymère de formule:

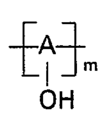

(dans laquelle A et *m* ont les mêmes significations que ci-dessus), la réaction de greffage étant effectuée au moyen d'initiateurs capables de former des radicaux libres à une température inférieure à 40°C; et à quaterniser les groupes basiques $R_1$ présents dans les copolymères greffés ainsi obtenus.

2. Procédé selon la revendication 1, dans lequel les initiateurs sont des systèmes redox choisis

dans le groupe comprenant $Fe^{++}/H_2O_2$; thiols/$H_2O_2$; $Na_2S_2O_3/H_2O_2$; $Ce^{+}$ en solution dans l'acide sulfurique ou nitrique; les sels de métaux de transition.

3. Procédé selon les revendications 1 et 2, dans lequel la quantité des chaînes greffées de formule

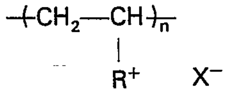

varie entre 10 et 60% du copolymère de formule (I) et leur poids moléculaire est compris entre 10.000 à 200.000.

4. Procédé selon les revendications 1 à 3 dans lequel A est le motif monomère de l'amidon.

5. Procédé selon les revendications 1 à 3, dans lequel A est le motif monomère d'hydroxyéthyl-cellulose.

6. Procédé selon les revendications 1 à 5, dans lequel R est

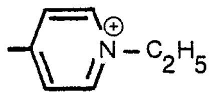

et X est $Cl^-$, $Br^-$ ou $I^-$.